# EUROPEAN PATENT APPLICATION

(11) **EP 1 002 878 A2**
(43) Date of publication of application: **24.05.2000**
(21) Application number: 99122092.2
(22) Date of filing: 18.11.1999
(51) Int. Cl.: C12Q 1/70, C12Q 1/68

(54) **Hepatitis C Virus RNA-binding oligo DNA and method preparing it**

(30) Priority: 19.11.1998 JP 32987498
(71) Applicant: Tosoh Corporation, Shinnanyo-shi, Yamaguchi-ken, 746-8501 (JP)
(72) Inventor: Toshiki, Taya, Sagamihara-shi, Kanagawa-ken (JP); Takahiko, Ishiguro, Yokohama-shi, Kanagawa-ken (JP); Juichi, Saitoh, Yamato-shi, Kanagawa-ken (JP)
(74) Representative: VOSSIUS & PARTNER

(57) **Abstract**

An HCV RNA-binding single-stranded oligo DNA of SEQ ID NO:1 which is complementary to nucleotides 101 to 120 of HCV RNA.

## Description

The present invention relates to a simple method of preparing a single-stranded oligo DNA which firmly binds to a single-stranded RNA and single-stranded oligo DNAs prepared by the method which firmly bind to HCV (hepatitis C virus) RNA. Oligo DNAs prepared by the method of the present invention are useful as reagents for gene diagnoses involving cleavage, amplification and detection of RNA and as reagents for inhibition of reverse transcription and translation of RNA. In particular, the sequence of a single-stranded oligo DNA prepared by the method of the present invention which firmly binds to HCV RNA is useful as a reagent used in diagnostic quantification of HCV RNA.

It is possible to know whether and, if any, how much HCV is present by detecting or quantifying HCV RNA. In quantification of a specific viral RNA, a single-stranded oligo DNA which complementarily binds to the RNA is used as a primer which serves as the starting point of reverse transcriptional DNA synthesis using the specific RNA as the template to form an RNA-DNA heteroduplex. Then, the DNA single strand in the RNA-DNA heteroduplex is denuded by the action of an enzyme which selectively cleaves the RNA strand in an RNA-DNA heteroduplex (a heteroduplexed RNA). Subsequently, an oligo DNA called a promoter primer, which has a region complementary to the DNA and a promoter region for RNA synthesis, coexisting with the DNA, elongates to form a double-stranded DNA having the above-mentioned promoter. Finally, an RNA polymerase acts on the double-stranded DNA to amplify the specific RNA used as the starting material, and then the RNA amplification product is detected or quantified (the RNA amplification continues by using the RNA amplification product as the starting material). Such a method can characteristically be performed at an almost constant temperature as compared with the DNA amplification method generally called PCR.

By virtue of the sequence specificity of hybridization of a nucleic acid sequence with a nucleic acid having a complementary nucleotide sequence (a nucleic acid probe), an oligo DNA which binds to a single-stranded RNA can be used not only for reverse transcription of the RNA in the above-mentioned amplification procedure but also as a probe for detection of the RNA amplification product and as an anti-RNA drug (which inhibits translation of an RNA by binding to the RNA) generally called an antisense drug. Further, not only an oligo DNA which binds to a specific RNA but also a DNA complementary to the complementary DNA, namely an oligo DNA containing a sequence in the specific RNA is essential in the above-mentioned RNA amplification.

An oligo DNA useful as a primer for the above-mentioned RNA amplification is obtainable by synthesizing a sequence complementary to the specific RNA as the target in accordance with the sequence (the primary structure) of the specific RNA. However, in many cases more information is needed in addition to its sequence.

Single-stranded RNA is known to partly have a three-dimensional structure due to intramolecular sequence-specific base-pairing between complementary regions. Therefore, it is necessary to design an oligo DNA complementary to a single-stranded region of RNA, namely a conformationally free region. Also, it is difficult to prepare a single-stranded oligo DNA which binds to a specific RNA when the sequence of the cDNA of the RNA is not known.

Of course, some approaches are available to estimate the three-dimensional structure of RNA. X-ray crystal analysis and NMR structural analysis can locate conformationally free regions in RNA. Conformationally free regions in a single-stranded RNA can be estimated from a computed molecular structure of the RNA. For experimental estimation of conformationally free regions, electrophoretic analysis of the fragments obtained by experimentation of cleavage using a single-stranded randomized oligo DNA of 10 or more nucleotides long and an enzyme RNaseH has been reported (J. Bio. Chem. 272, 626-638, 1997, etc.).

However, even though various known approaches are available for estimation, analytic approaches are practically inapplicable to viral RNA samples which are usually several hundred nucleotides long because an analysis of a single-stranded RNA of even several ten nucleotides long consumes a lot of time and labor. Computational estimation of conformationally free regions is based on a computed two-dimensional (secondary) structure of a single-stranded RNA, but quite a few computed two-dimensionally free regions are not actually free because in a liquid phase, complementary regions of an RNA molecule which are remote from the aspect of the secondary structure but spatially close from the aspect of the actual three-dimensional (tertiary) structure can sequence-specifically bind to each other. In fact, it can happen so often that even if a single-stranded RNA is treated with an oligo DNA having a sequence complementary to a computed two-dimensionally free region, the oligo DNA does not bind firmly.

On the other hand, the approach using a randomized oligo DNA and RNaseH, which selectively cleaves a heteroduplexed RNA, is considered to give more accurate results than the computational approach because it reflects the three-dimensional structure of a single-stranded RNA in solution to be treated with RNaseH. However, it has been reported so far that a library of a randomized nucleic acid of at least 10 nucleotides long and analysis of the results of cleavage reaction for various sequences as many as over a million are necessary. Therefore, a high concentration library solution is indispensable. Further, because one RNA molecule can form more than one duplex with a randomized oligo DNA to be used and develop several cleavages by the action of the enzyme, there is a problem that it is virtually impossible to judge whether these cleavages are attributed to their firm binding or unfavorably to their weak binding. In addition, a longer oligo DNA is generally more difficult to randomize evenly. Further, because a longer oligo DNA is more likely to direct a specific RNA to cleavage at conformationally free regions even if their binding is relatively weak and thereby leads to production of a larger number of cleavage fragments, it is difficult to select a proper oligo DNA which binds to a specific RNA from the library. Therefore, in conclusion, this approach is not suitable for production of a single-stranded DNA which firmly binds to a specific RNA even though useful to examine whether or not these random sequences bind to the specific RNA.

As described above, currently used oligo DNAs which bind to a specific RNA are prepared so as to have a sequence complementary to an estimated conformationally free region of the specific RNA or obtained by screening various oligo DNAs containing a sequence complementary to the region.

Accordingly, the object of the present invention is to provide a simple method of preparing a single-stranded oligo DNA useful as a reagent for gene diagnoses involving cleavage, amplification and detection of RNA or as an inhibitory drug against reverse transcription or translation of RNA, and in particular to provide single-stranded oligo DNAs which firmly bind to HCV RNA prepared by the method.

Namely, the present invention provides an HCV RNA-binding single-stranded oligo DNA of SEQ ID NO:1 which is complementary to nucleotides 101 to 120 of HCV RNA, an HCV RNA-binding single-stranded oligo DNA of SEQ ID NO:2 which is complementary to nucleotides 99 to 118 of HCV RNA, an HCV RNA-binding single-stranded oligo DNA of SEQ ID NO:3 which is complementary to nucleotides 97 to 116 of HCV RNA, an HCV RNA-binding single-stranded oligo DNA of SEQ ID NO:4 which is complementary to nucleotides 95 to 114 of HCV RNA, an HCV RNA-binding single-stranded oligo DNA of SEQ ID NO:5 which is complementary to nucleotides 248 to 267 of HCV RNA or an HCV RNA-binding single-stranded oligo DNA of SEQ ID NO:6 which is complementary to nucleotides 238 to 257 of HCV RNA.

The present invention also provides a single-stranded oligo DNA of SEQ ID NO:7 which is useful as a sense primer or a promoter primer and is identical to nucleotides 113 to 137 of HCV RNA, a single-stranded oligo DNA of SEQ ID NO:8 which is useful as a sense primer or a promoter primer and is identical to nucleotides 115 to 139 of HCV RNA or a single-stranded oligo DNA of SEQ ID NO:9 which is useful as a sense primer or a promoter primer and is identical to nucleotides 111 to 135 of HCV RNA.

The present invention still further provides a method of preparing a single-stranded oligo DNA which is complementary to a conformationally free region in a single-stranded RNA and therefore firmly binds to the single-stranded RNA, which comprises estimating a cleaving site in the single-stranded RNA by experimenting with cleavage reactions using a randomized single-stranded oligo DNA of 6 to 9 nucleotides long and an enzyme which selectively cleaves a heteroduplexed RNA and screening a multiple oligo DNA set of 20 to 25 nucleotides long containing a sequence complementary to the estimated cleaving site or its vicinity by experimenting with cleavage reactions using the enzyme which selectively cleaves a heteroduplexed RNA.

The present invention further provides a reagent for cleavage of a single-stranded RNA, amplification of a single-stranded RNA, reverse transcription of a single-stranded RNA, inhibition of translation of a single-stranded RNA or assay of a single-stranded RNA, which is part of the single-stranded oligo DNA of any one of SEQ ID NOS: 1 to 6, the whole of the single-stranded oligo DNA of any one of SEQ ID NOS: 1 to 6, part of the single-stranded oligo DNA selected by the above-mentioned method, the whole of the single-stranded oligo DNA selected by the above-mentioned method or a derivative thereof.

The present invention also provides a reagent for cleaving a single-stranded RNA, which comprises an RNA-cleaving motif and part of the single-stranded oligo DNA of any one of SEQ ID NOS: 1 to 6, the whole of the single-stranded oligo DNA of any one of SEQ ID NOS: 1 to 6, part of the single-stranded oligo DNA selected by the above-mentioned method, the whole of the single-stranded oligo DNA selected by the above-mentioned method or a derivative thereof to the RNA-cleaving motif.

The present invention still further provides a reagent as a sense primer or promoter primer for a single-stranded RNA, which is a single-stranded oligo DNA complementary to part of the single-stranded oligo DNA of any one of SEQ ID NOS: 1 to 6, the whole of the single-stranded oligo DNA of any one of SEQ ID NOS: 1 to 6, part of the single-stranded oligo DNA selected by the above-mentioned method or the whole of the single-stranded oligo DNA selected by the above-mentioned method or a derivative thereof.
Fig. 1 shows the gel electrophoresis pattern obtained by cleavage of HCV RNA of about 1500 nucleotides long having an isotopically labeled 5' end by RNaseH in the presence of a randomized single-stranded oligo DNA of 6 nucleotides long. The two arrows indicate clear bands.
Fig. 2 shows the gel electrophoresis pattern obtained by another cleavage of HCV RNA in the presence of single-stranded oligo DNAs prepared on the basis of the results in Example 1. Clear bands developed in all lanes except the rightmost two lanes.
Fig. 3 show the gel electrophoresis pattern obtained by cleavage of HCV RNA by AMV-RTase in the presence of single-stranded oligo DNAs prepared on the basis of the results in Examples 1 and 2. Bands developed even when AMV-RTase was used instead of RNaseH, and clear bands developed with any of the newly prepared single-stranded oligo DNAs.
Fig. 4 shows the gel electrophoresis pattern obtained by cleavage of HCV RNA in the presence of a single-stranded oligo DNA linked to a cleaving motif. In the presence of a cleaving motif like a DNAzyme, bands attributable to the product of specific cleavage developed at the position indicated by the arrow.
Fig. 5 shows the results of amplification of HCV RNA using a single-stranded oligo DNA of the present invention. In most cases, the amplification product developed as bands at the position indicated by the arrow.

Now, the present invention will be described in detail. Herein, the nucleotides in HCV RNA are numbered in accordance with the numbers of nucleotides in HCV cDNA used in Kato et al. (Proc. Natl. Acad. Sci. USA, 87, 9524-9528, 1990)

### 1. HCV RNA-binding single-stranded oligo DNAs

These single-stranded oligo DNAs are prepared by the after-mentioned method of the present invention and complementarily bind to conformationally free regions in HCV RNA.

The single-stranded oligo DNA of SEQ ID NO: 1 binds to HCV RNA with high binding ability and is complementary to nucleotides 101 to 120 of HCV RNA. The single-stranded oligo DNA of SEQ ID NO: 2 binds to HCV RNA with high binding ability and is complementary to nucleotides 99 to 118 of HCV RNA. The single-stranded oligo DNA of SEQ ID NO: 3 binds to HCV RNA with high binding ability and is complementary to nucleotides 97 to 116 of HCV RNA. The single-stranded oligo DNA of SEQ ID NO: 4 binds to HCV RNA with high binding ability and is complementary to nucleotides 95 to 114 of HCV RNA. The single-stranded oligo DNA of SEQ ID NO: 5 binds to HCV RNA with high binding ability and is complementary to nucleotides 248 to 267 of HCV RNA. The single-stranded oligo DNA of SEQ ID NO: 6 binds to HCV RNA with high binding ability and is complementary to nucleotides 238 to 257 of HCV RNA.

These HCV RNA-binding single-stranded oligo DNAs are useful as reagents for cleavage, amplification, reverse transcription, inhibition of translation or assay of a single-stranded RNA by themselves. Namely, cleavage of single-stranded HCV RNA is accomplished by formation of a RNA-DNA heteroduplex between the RNA and them followed by treatment with an enzyme which selectively cleaves a heteroduplexed RNA such as RNaseH. As RNaseH, avian myoblastoma virus (AMV) reverse transcriptase (AMV-RTase) may be mentioned. Otherwise, an RNA-cleaving motif which cleaves RNA non-specifically may be linked to them.

For the purpose of HCV RNA amplification, the single-stranded oligo DNAs of the present invention may be used in the above-mentioned RNA amplification procedure as a primer which directs cDNA synthesis by reverse transcription of a specific RNA. In the above-mentioned RNA amplification procedure, the single-stranded oligo DNAs of the present invention may be used as a promoter primer for RNA reverse transcription after linked to a promoter for RNA synthesis to amplify an RNA complementary to HCV RNA. Further, the single-stranded oligo DNAs of the present invention can be used to cleave the 5' end of HCV RNA at an arbitrary site prior to the amplification procedure. In addition to amplification, they may be used for simple reverse transcription of HCV RNA. They may also be used as a primer in PCR of a double-stranded DNA having a DNA strand complementary to HCV RNA obtained by reverse transcription.

The single-stranded oligo DNAs of the present invention may be used as an antisense drug to inhibit translation of HCV RNA.

For assay of HCV RNA, the single-stranded oligo DNAs of the present invention may be used as a probe.

In the above-mentioned applications, the sequences of the single-stranded oligo DNAs of the present invention may be used entirely or partly. In the former case, a sequence containing the entire sequence of a single-stranded oligo DNA of the present invention may be used. In the latter case, a sequence of at least 5 consecutive nucleotides in any of SEQ ID NOS: 1 to 6 is preferably used to secure the specificity and binding ability for HCV RNA. Derivatives of the single-stranded oligo DNAs of the present invention may also be used similarly. Examples of these derivatives include those obtained by replacement of the phosphorus atoms in DNA molecules by sulfur atoms or replacement of some deoxyribose moieties in DNA molecules by deoxyribose moieties for improved degradation resistance in blood as well as those obtained by linking an RNA-cleaving motif or by linking a label such as an enzyme, a light absorptive substance, a luminescent substance, a fluorescent substance, a radioisotope or a fluorescent intercalative dye for use as a probe, if necessary, via a linker having an appropriate functional group.

### 2. Single-stranded oligo DNAs identical to sequences in HCV RNA

These single-stranded oligo DNAs are used usefully especially as a sense primer for HCV RNA, which directs a sequence complementary to HCV RNA to be amplified in DNA or RNA, and as a reagent called a promoter primer in the above-mentioned amplification procedure for HCV RNA after linked to a known promoter region (sequence) for RNA synthesis.

The single-stranded oligo DNA of SEQ ID NO:7 is identical to the sequence of nucleotides 113 to 137 of HCV RNA. The single-stranded oligo DNA of SEQ ID NO:8 is identical to the sequence of nucleotides 115 to 139 of HCV RNA. The single-stranded oligo DNA of SEQ ID NO:9 is identical to the sequence of nucleotides 111 to 135 of HCV RNA.

These single-stranded oligo DNAs identical to sequences in HCV RNA may be used not only as a sense primer or a promoter primer but also as a cleaving reagent to cleave a single-stranded RNA containing a sequence complementary to HCV RNA. In such a case, they form an RNA-DNA heteroduplex with the RNA, and the RNA strand in the heteroduplex is selectively cleaved by an enzyme such as RNaseH. In addition, they may be liked to an RNA-cleaving motif which directs non-specific cleavage of RNA. Still further, they may be used as a probe to detect a sequence complementary to HCV RNA in DNA or RNA.

In the above-mentioned applications, the sequences of the single-stranded oligo DNAs identical to sequences in HCV RNA may be used entirely or partly. In the former case, a sequence containing the entire sequence of a single-stranded oligo DNA of the present invention may be used. In the latter case, a sequence of at least 5 consecutive nucleotides in any of SEQ ID NOS: 7 to 9 is preferably used to secure the specificity and binding ability for HCV RNA. Derivatives of the single-stranded oligo DNAs of the present invention may also be used similarly. Examples of these derivatives include those obtained by replacement of the phosphorus atoms in DNA molecules by sulfur atoms or replacement of some deoxyribose moieties in DNA molecules by deoxyribose moieties for improved degradation resistance in blood as well as those obtained by linking an RNA-cleaving motif or by linking a label such as an enzyme, a light absorptive substance, a luminescent substance, a fluorescent substance, a radioisotope or a fluorescent intercalative dye for use as a probe, if necessary, via a linker having an appropriate functional group.

### 3. Simple method of preparing a single-stranded oligo DNA which firmly binds to a single-stranded RNA

The method of the present invention of preparing a single-stranded oligo DNA which firmly binds to a single-stranded RNA can be used preferably for an RNA having a known primary structure (or an RNA whose cDNA has a known primary structure because the primary structure of an RNA is naturally obvious from the primary structure of its cDNA) such as HCV or HIV RNA, though it may be used for an isolated single-stranded RNA whose primary structure is not known.

The first step in the method of the present invention is preparation of a randomized single-stranded oligo DNA of about 6 nucleotides long. The length is preferably 6 nucleotides because DNAs shorter than that can cause a problem of formation of quite a few cleavages in the RNA, like DNAs of at least 10 nucleotides long, by binding to infinite sequences in the RNA. The randomized single-stranded oligo DNA is used as a probe at nearly physiological low temperatures to explore conformationally free regions, namely regions which form no higher order structure, in a single-stranded RNA. Because there are four DNA-constituting bases, adenine, guanine, cytosine and thymine, 4096 different sequences are possible for 6 nucleotides. Although there is no need to prepare all these sequences, a library of single-stranded oligo DNAs of all the possible sequences makes the method of the present invention applicable to any single-stranded RNA. They are obtainable by using an automatic DNA synthesizer or as commercial random sequences.

On the other hand, for an RNA having a known primary structure, a set of single-stranded oligo DNAs complementary to successive nucleotide sequences in any specific region of the RNA may be used as a randomized oligo DNA in the method of the present invention. However, such an oligo DNA set lacks versatility, and a new single-stranded oligo DNA set has to be prepared every time the method of the present invention is applied to a different RNA. Besides, if an oligo DNA set is found to lack enough binding ability or contain many nucleotides which do not contribute to hybridization with the RNA due to the conformation of the RNA in the after-mentioned course of the method of the present invention, despite the complementarity from the aspect of the primary structure, a different oligo DNA set has to be prepared anew. Further, such a oligo DNA set can not locate more than one conformationally free region in a single-stranded RNA at a time. Therefore, the above-mentioned randomized single-stranded oligo DNA is preferably used in the method of the present invention.

A randomized single-stranded oligo DNA thus prepared is allowed to hybridize with a single-stranded RNA modified with a detectable label at the 5' end or 3' end to form an RNA-DNA heteroduplex, and then an enzyme which selectively cleaves a heteroduplexed RNA acts on it. As the enzyme, an enzyme known to have an RNaseH activity, such as avian myoblastoma virus (AMV) reverse transcriptase (AMV-RTase), is usually used. For the modification of a single-stranded RNA, known methods such as isotopic labeling of the 5' end with [γ-³²P]ATP may be employed.

A randomized single-stranded oligo DNA is used in large excess in relation to the RNA, preferably at a concentration of from 0.5 mM to 5 mM because this concentration range enables assessment of all the single-stranded oligo DNA sequences at once in a case of an RNA having a known primary structure. Because it is expected that quite a few cleavages are formed enzymatically in a single-stranded RNA where the single-stranded RNA is conformationally free due to putative formation of duplexes with single-stranded oligo DNAs, the enzymatic cleavage reaction should be conducted with an enzyme concentration and a reaction time suitable for the after-mentioned analysis. In a case of a single-stranded RNA having an unknown primary structure, the enzymatic treatment is conducted separately for each sequence of the randomized single-stranded oligo DNA to give the results to be analyzed.

Then, the products of the enzymatic cleavages, namely the fragments of the cleaved RNA, are analyzed by an analytic molecular sizing available for estimation of the lengths of RNA fragments such as denaturing polyacrylamide gel electrophoresis. The gel used for electrophoresis is preferably several ten cm long to secure a long migration distance, but may be about 15 cm long for the sake of simplicity. Electrophoretic analysis is explained as an example. The RNA fragments as the products of the enzymatic cleavage reaction migrate in the gel according to their lengths and develop bands. With a calibration curve which correlates the lengths of RNA markers of known lengths and their migration distances in electrophoresis under the same conditions, the lengths of the cleavage products can be estimated from their actual migration distances, and the location of cleavages in the single-stranded RNA can be estimated through detection of the label at the 5' or 3' end of the single-stranded RNA.

When the enzymatic cleavage of the RNA yields no appropriate cleavage products, it is advisable to elongate a randomized single-stranded oligo DNA by one-by-one addition of nucleotides for preparation of a single-stranded oligo DNA having high binding ability. Because DNAs of 10 or more nucleotides long make it difficult to prepare a single-stranded oligo DNA having high binding ability due to the conformational competence of their own and the increasingly uneven randomization, it is preferred to use DNAs of at most about 9 nucleotides long, particularly at most about 8 nucleotides long.

The next step is preparation of sequences complementary to the vicinity of the estimated cleaving sites for use in the following step as the candidates for the single-stranded oligo DNA which binds to a conformationally free region in the single-stranded RNA. In order to control the number of the single-stranded oligo DNAs to be prepared as the candidates within the range from 3 to 10, it is preferred to so adjust the enzyme concentration and reaction time in the preceding enzymatic RNA cleavage that 3 to 10 bands develop in the electrophoretic analysis of the cleavage products. Of course, it is preferable to prepare as many candidates as possible, but the number of candidates to be actually prepared should be limited to about 10 for the sake of simplicity and speed of the following operations. If no particular limitations are placed on the enzyme concentration and the like, because the number of bands, namely RNA fragments as the cleavage products increases, there is a problem of the difficulty in the screening of candidates. However, the screening may be based on the band density (darkness). When the enzymatic cleavage of a single-stranded RNA having an unknown sequence is analyzed separately for each sequence of a randomized single-stranded oligo DNA as described above, all the operations should be carried out with the same enzyme concentration and reaction time, and it is preferred to select sequences which lead to development of dense bands as candidates.

Single-stranded oligo DNAs as the candidates mentioned above can be prepared, for example, as follows. Firstly, their length should be determined. There is no particular restriction on the length as long as it is greater than the length of the previously used randomized single-stranded oligo DNA of about 6 nucleotides long. However, in a case of a single-stranded RNA having a known primary structure, the length may be increased in accordance with the known primary structure, if necessary, for the sake of not only biding ability but also binding specificity. In such a case, single-stranded oligo DNAs of 20 to 25 nucleotides long usually have excellent binding specificity and are more likely to cover a conformationally free region. Specifically speaking, once the length is determined, a single-stranded DNA of the predetermined length which has a sequence complementary to a sequence centered at the estimated cleaving site in a single-stranded RNA is preferable as the first candidate. As the second candidate, a single-stranded oligo DNA of the predetermined length which has a sequence complementary to a sequence centered half the length upstream of the estimated cleaving site in the RNA is preferable. As the third candidate, a single-stranded oligo DNA of the predetermined length which has a sequence complementary to a sequence centered half the length downstream of the estimated cleaving site in the RNA is preferable.

Once these three single-stranded oligo DNAs having the specific sequences are prepared as the candidates, they are subjected to another enzymatic treatment similar to that described previously and checked for formation of duplexes with the single-stranded RNA, for example, by determining the migration distances of the bands attributable to the RNA cleavage products in denaturing polyacrylamide gel electrophoresis. It is not necessary to label either end of the single-stranded RNA in this step, and a gel of about 10 cm long is satisfactory for electrophoretic analysis. If there are any bands attributable to cleavage products, their band densities are compared, and a single-stranded oligo DNA which develops a denser band is considered to have higher binding ability for the single-stranded RNA. If no cleavage products are obtained with any candidate single-stranded oligo DNA, the above-mentioned procedure is repeated to explore other candidates until a single-stranded RNA-binding single-stranded oligo DNA having high binding ability is obtained finally.

Single-stranded oligo DNAs prepared by the above-mentioned method which bind to HCV RNA or other RNAs and are complementary to specific sequences in these RNAs are useful as reagents for cleavage, amplification, reverse transcription, inhibition of translation or assay of a single-stranded RNA, like the single-stranded oligo DNAs of SEQ ID NOS: 1 to 6 of the present invention. In these applications, the sequence of a single-stranded oligo DNA prepared by the method of the present invention may be used entirely or partly. In the latter case, it is preferred to use a sequence of at least 5 consecutive nucleotides in it. Further, a single-stranded oligo DNA prepared by the method of the present invention may be converted into a derivative, for example, by replacing the phosphorus atoms in DNA molecules with sulfur atoms, replacing some deoxyribose moieties in DNA molecules with deoxyribose moieties or linking an RNA-cleaving motif or a label.

A single-stranded oligo DNA complementary to an RNA-binding single-stranded oligo DNA which has high binding ability for a specific RNA such as HCV RNA and is complementary to a specific sequence in the specific RNA, namely, a single-stranded oligo DNA identical to a specific sequence in the specific RNA, is also useful as a sense primer or promoter primer for the specific RNA, as described above for the single-stranded oligo DNAs of SEQ ID NOS:7 to 9 of the present invention. In such a case, the sequence of the single-stranded oligo DNA may be used entirely or partly. In the latter case, it is preferred to use a sequence of at least 5 consecutive nucleotides in it. Further, such a sequence may be converted into a derivative, for example, by replacing the phosphorus atoms in DNA molecules with sulfur atoms, replacing some deoxyribose moieties in DNA molecules with deoxyribose moieties or linking an RNA-cleaving motif of a label.

Now, the oresent invention will be described in further detail by referring to Examples. However, the present invention is by no means restricted to these specific Examples.

### EXAMPLE 1

HCV RNA was cleaved by using a randomized single-stranded oligo DNA of 6 nucleotides long in accordance with the following procedure to estimated cleaving sites in it.
1. 1.5 µℓ of a reaction solution having the following composition was dispensed into 1.5 mℓ tubes.
   200 mM Tris-HCℓ buffer (pH 7.5)
   200 mM Potassium chloride
   100 mM Magnesium chloride
   1 mM EDTA
   1 mM Dithiothreitol (DTT)
2. 7.5 µℓ of a standard HCV RNA isotopically labeled with [γ-³²P]ATP at the 5' end (10¹¹ copies/µℓ) was added to the tubes. The standard HCV RNA was 1549 nucleotides long, with a 5'-end 62-nucleotide vector sequence and nucleotides 1 to 1487 of HCV type II.
3. 5 µℓ of a randomized single-stranded oligo DNA of 6 nucleotides long a random sequence (Takara Shuzo, 1.5 mM) was added.
4. 1 µℓ of an RNaseH (Takara Shuzo, 0.1 U/µℓ) was added.
5. Reaction was conducted at 37°C for 5 to 15 minutes.
6. An 7.5 µℓ aliquot from each reaction solution was subjected to electrophoresis on a 5% polyacrylamide gel containing 7M urea (15 cm long). As the marker, a commercially available marker (trade name: perfect RNA Marker, Novagen) was used after isotopically labeled with [γ-³²P]ATP at the 5' end.
7. After the electrophoresis, the gel was dried at 60°C for 2 hours under aspirated reduced pressure.
8. An X-ray film was exposed to the gel to develop bands.

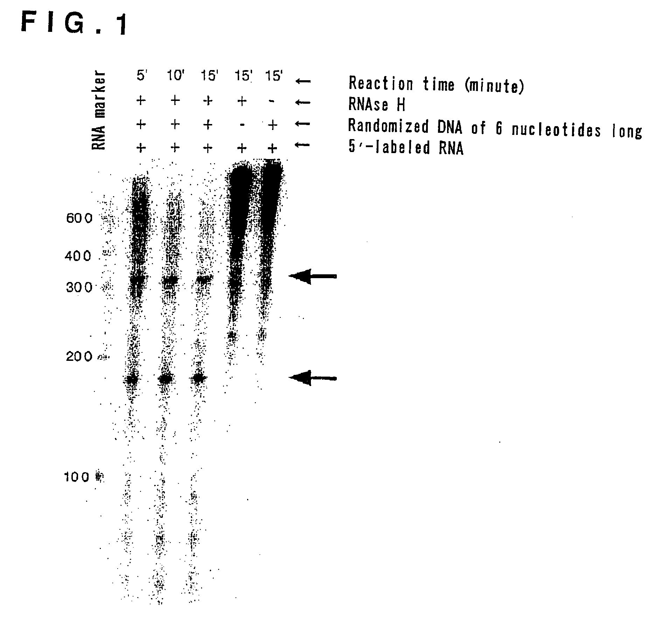

Fig. 1 shows the pattern obtained on the X-ray film. As shown in Fig. 1, clear and dense bands attributed to the abundant individual cleavage products were detected between the 400-nucleotide marker and the 300-nucleotide marker and between the 200-nucleotide marker and the 100-nucleotide marker. The migration distances of the markers and their lengths were correlated on a calibration curve, and the cleavage products were determined to be 309 and 162 nucleotides long from the calibration curve. The results of this Example suggest that HCV RNA cleaved at around the 310th nucleotide and at the 160th nucleotide from the 5' end, namely that HCV RNA was conformationally free around these cleaves.

### EXAMPLE 2

The following procedure was carried out to find a single-stranded oligo DNA sequence which leads to cleavage of single-stranded HCV RNA in the presence of RNaseH by firmly binding to it.
1. 5.2 µℓ of a reaction solution having the following composition was dispensed into 1.5 mℓ tubes.
   200 mM Tris-HCℓ (pH 7.5)
   200 mM Potassium chloride
   100 mM Magnesium chloride
   1 mM EDTA
   1 mM DTT
2. 3.8 µℓ of a standard HCV RNA (2×10¹¹ copies/µℓ) was added to the tubes.
3. Single-stranded oligo DNAs of 20 nucleotides long complementary to sequences in HCV RNA centered at each cleaving site estimated in Example 1, or 10 nucleotides upstream or downstream of each cleaving site were prepared, six in total, three DNAs for each cleave (these oligo DNAs were complementary to nucleotides 248 to 267, nucleotides 238 to 257, nucleotides 228 to 247, nucleotides 101 to 120, nucleotides 91 to 110 and nucleotides 81 to 100 in HCV RNA). These synthetic DNAs (0.25 µM) were added to the tubes, 5 µℓ each, and the tubes were subjected to the following treatments together with control tubes to which 5 µℓ of a solution containing no single-stranded DNA or a solution of a randomized single-stranded oligo DNAs of 6 nucleotides long (Takara Shuzo, 1.5 mM) was added.24
4. 1 µℓ of RNaseH (Takara Shuzo, 0.1 U/µℓ) was added to the tubes.
5. Reaction was conducted at 37°C for 15 minutes.
5. A 7.5 µℓ aliquot from each reaction solution was subjected to electrophoresis on a 5% polyacrylamide gel containing 7M urea (10 cm long).
6. After the electrophoresis, the gel was stained with a 10000-fold diluted stain (Takara Shuzo, trade name: SYBR Green II) for 30 minutes.
7. The staining pattern was observed on a transilluminator.

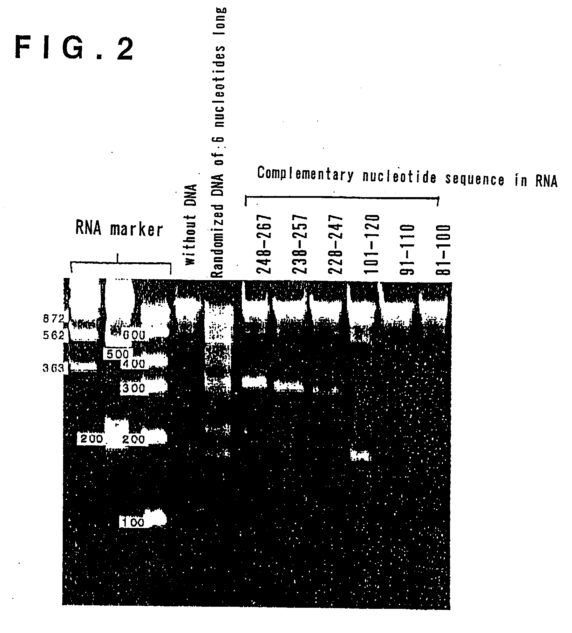

Fig. 2 shows the staining pattern on the gel. In Fig. 2, bands attributable to cleavage products were obtained with four among the randomized DNA of 6 nucleotides long and the six synthetic single-stranded oligo DNAs. The bands had various densities (darknesses), and denser bands were obtained with the DNAs complementary to nucleotides 248 to 267, nucleotides 238 to 257 and nucleotides 101 to 120 than with the randomized DNA of 6 nucleotides long. The lengths of the cleavage products developed as these bands (when more than one band was obtained, the length of the cleavage product corresponding to the denser band) corresponded to the lengths of the 5'-end fragments of the RNA produced by the cleavage of the RNA resulting from binding of the synthetic single-stranded oligo DNAs to the estimated cleaving sites in HCV RNA. The results of this Example demonstrate that single-stranded oligo DNAs of 20 nucleotides long which cleaves HCV RNA by binding to vicinities of the cleaving sites in HCV RNA estimated by using a randomized single-stranded oligo DNAs of 6 nucleotides long and RNaseH more firmly than the randomized single-stranded oligo DNAs of 6 nucleotides long were prepared. The regions in HCV RNA complementary to the single-stranded oligo DNAs which lead to development of dense bands in this Example are supposed to be conformationally free and agree with the conformationally free regions estimated in Example 1.

### EXAMPLE 3

The sequences of the single-stranded oligo DNA probes which lead to development of dense bands in Example 2 were modified by the following procedure and used for investigation of cleavage of HCV RNA and for demonstration of usefulness of reverse transcriptase AMV-RTase as the enzyme used for cleavage.
1. 0.75 µℓ of a reaction solution having the following composition was dispensed into 1.5 mℓ tubes.
   400 mM Tris-acetate (pH 8.1)
   1250 mM Potassium acetate
   140 mM Magnesium acetate
   100 mM DTT
   1 mg/mℓ Bovine serum albumin (BSA)
   20 U/µℓ RNase inhibitor
2. 3.5 µℓ of a standard HCV RNA (8.1×10¹¹ copies/µℓ) was added to the tubes.
3. Single-stranded oligo DNAs complementary to the sequence of nucleotides 101 to 120 in HCV RNA and sequences in HCV RNA 2, 4 and 6 nucleotides upstream, namely nucleotides 99 to 118, nucleotides 97 to 116 and nucleotides 95 to 114, were prepared. 2.5 µℓ of these four synthetic DNAs (2 µM) and the single-stranded oligo DNAs (2 µM) complementary to nucleotides 81 to 100 and nucleotides 91 to 110 used in Example 2 were added, 2.5 µℓ each. The tubes were subjected to the following treatments together with a tube for the control reaction to which a solution containing no DNA added.
4. 0.75 µℓ of AMV-RTase (Takara Shuzo, 14 U/µℓ) was added.
5. Reaction was conducted at 37°C for 20 minutes.
6. The entire volume of each reaction solution was subjected to electrophoresis on a 5% polyacrylamide gel containing 7M urea (10 cm long).
7. After the electrophoresis, the gel was stained with a 1000-fold diluted stain (Takara Shuzo, trade name: SYBR Green II) for 30 minutes.
8. The staining pattern was observed on a transilluminator.

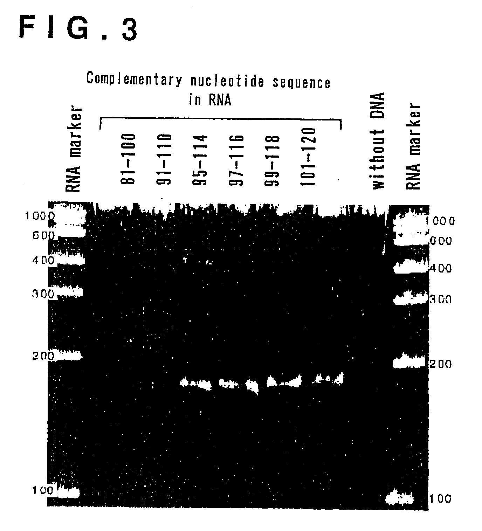

Fig. 3 shows the staining pattern on the gel. In Fig.3, bands attributable to cleavage products were obtained with most of the single-stranded oligo DNAs. The bands had various densities (darknesses), and the bands obtained with the oligo DNAs complementary to the sequence of nucleotides 101 to 120 and the sequences 10 and 20 nucleotides upstream in HCV RNA (nucleotides 81 to 100 and nucleotides 91 to 110) were as dense as those obtained in Example 2. The bands developed with single-stranded oligo DNAs complementary to nucleotides 99 to 118, nucleotides 97 to 116 and nucleotides 95 to 114 were about the same in density. It was demonstrated by determination of the migration distances of the bands that as the sequence in HCV RNA complementary to a single-stranded oligo DNA shifted upstream, the RNA fragment as the cleavage product became shorter.

The results of this Example demonstrate that AMV-RTase was useful as a cleaving reagent having the cleaving activity similarly to RNaseH. Further, it was indicated that the band density of a single-stranded oligo nucleotide which lead to development of a dense band in Example 2 remained almost unchanged even if the complementary sequence in HCV RNA was shifted 2 or 6 nucleotides upstream. These results indicate that HCV-binding single-stranded oligo DNAs which bind to HCV RNA with high binding ability were effectively prepared by modifications to the three single-stranded oligo DNAs prepared in Example 2 which shifted the complementary sequence in HCV RNA upstream or downstream.

### EXAMPLE 4

An RNA was cleaved by using single-stranded oligo DNAs linked to a RNA-cleaving DNA sequence motif without using RNaseH or any other enzymes in accordance with the following procedure.
1. 1.5 µℓ of a reaction solution having the following composition was dispensed into 1.5 mℓ tubes.
   500 mM Tris-HCℓ (pH 7.5)
   10 mM DTT
   10 U/µℓ RNase inhibitor
2. 1.5 µl of a standard HCV RNA (4.7×10¹¹ copies/µℓ) was added to the tubes.
3. A single-stranded oligo DNA complementary to nucleotides 99 to 106 in HCV RNA, an RNA-cleaving DNA sequence motif (Proc. Natl. Acad. Sci. USA, 94, 4262-4266 (1997)) and a single-stranded oligo DNA complementary to nucleotides 108 to 115 in HCV RNA were linked in this order from the 5' end, and 1.5 µℓ of the resulting synthetic DNA (2 µM) in solutions of various magnesium chloride concentrations was added. The sequence of the synthetic DNA was shown in SEQ ID NO:10.
4. Distilled water was added to a total volume of 15 µℓ.
5. Reaction was conducted at 37°C for 120 minutes.
6. The entire volume of each reaction solution was subjected to electrophoresis on a 5% polyacrylamide gel containing 7M urea (10 cm long).
7. After the electrophoresis, the gel was stained with a 10000-fold diluted stain (Takara Shuzo, trade name: SYBR Green II) for 30 minutes.
8. The staining pattern was observed on a transilluminator.

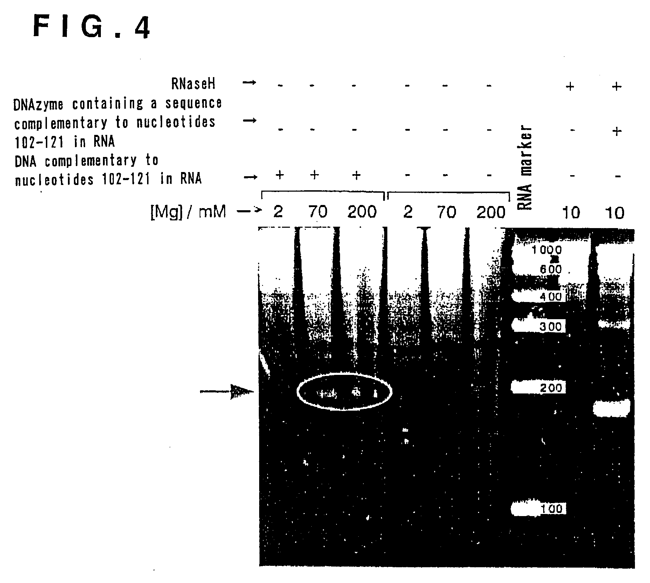

Fig. 4 shows the staining pattern on the gel. As the results obtained with various magnesium chloride concentrations 2, 70 and 200 mM shown in Fig. 4 indicate, due to the magnesium ion dependency of the RNA cleaving activity of the RNA-cleaving DNA motif, bands developed in the presence of at least 70 mM of magnesium ion, like in the presence of RNaseH or AMV-RTase. The bands were at the same position as the band developed by using a single-stranded oligo DNA complementary to nucleotides 101 to 120 in HCV RNA.

The results of this Example indicate that the single-stranded oligo DNA bound to a conformationally free region in the RNA, and HCV RNA was cleaved at the region by the action of the cleaving motif in the DNA.

### EXAMPLE 5

HCV RNA was amplified by using a single-stranded oligo DNA of the present invention in accordance with the following procedure.
1. 18 µℓ of a reaction solution having the following composition was dispensed into twelve PCR tubes.
   68 mM Tris-HCℓ (pH 8.1)
   210 mM Potassium acetate
   23 mM Magnesium acetate
   27% Sorbitol
   5.1% DMSO
   17 mM DTT
   170 µg/mℓ BSA
   3.4 U/µℓ RNase inhibitor
   2.3/µℓ AMV-RTase
2. The reaction solutions were overlaid with 80 µℓ of mineral oil.
3. 0.6 µℓ of a single-stranded oligo DNA complementary to nucleotides 95 to114 in HCV RNA, which was demonstrated in Example 3 to bind firmly to HCV RNA (with a 3'-end amino modification which prevents elongation of the 3' end of the sequence in the subsequent amplification) was added.
4. A standard HCV RNA (3.8×10⁵ copies/µℓ) as a positive solution was added in duplicate, and TE buffer as a negative solution was added singularly, 4.4 µℓ each.
5. Reaction was conducted at 37°C for 120 minutes.
6. Heating was conducted at 50°C for 5 minutes.
7. 5.5 µℓ of reaction solutions containing different promoter primers having the following composition were added.
   31 U/µℓ SP6-RNA polymerase
   5.5 mM each of dATP, dGTP, dCTP and dTTP
   1.1 µM antisense primer (single-stranded oligo DNA of SEQ ID NO:5 complementary to nucleotides 248 to 267 in HCV RNA)
   1.1 µM promoter primers
   of SEQ ID NO:11 containing the sequence of nucleotides 113 to 137 in HCV RNA (SEQ ID NO:7)
   of SEQ ID NO:12 containing the sequence of nucleotides 115 to 139 in HCV RNA (SEQ ID NO:8)
   of SEQ ID NO:13 containing the sequence of nucleotides 117 to 141 in HCV RNA, and
   of SEQ ID NO:14 containing the sequence of nucleotides 111 to 135 in HCV RNA (SEQ ID NO:9)
8. Reaction was conducted at 50°C for 10 minutes.
9. 20 mM of ATP, GTP, CTP and TTP were added each, 1.5 mℓ in total.
10. Reaction was conducted at 50°C for 2 hours.
11. A 5 µℓ aliquot from each reaction solution was subjected to 4% agarose gel electrophoresis (on a gel of 5 cm long).
12. After the electrophoresis, the gel was stained with a 10000-fold diluted stain (Takara Shuzo, trade name: SYBR Green II) for 30 minutes.
13. The staining pattern was observed on a transilluminator.

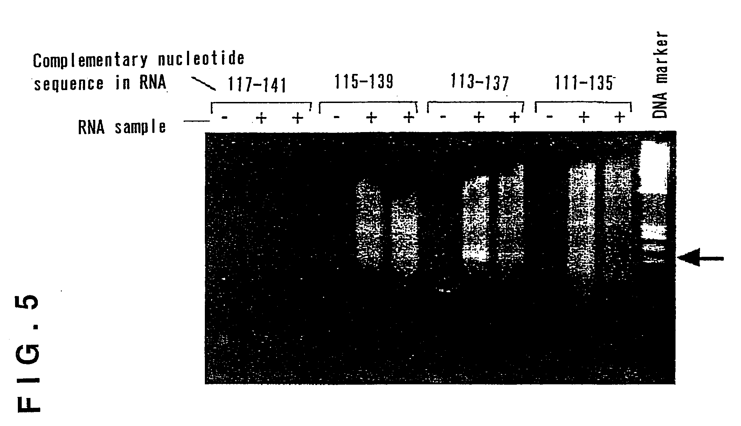

Fig. 5 shows the staining pattern on the gel. The negative sample gave no HCV RNA amplification product identified as a band irrespective of the kind of the promoter primer used, while the positive sample gave a HCV RNA amplification product as a band.

The band obtained with the positive sample in this Example corresponds to the amplification product of the of HCV RNA using the single-stranded oligo DNA with a 3'-end amino modification of the present invention designed for beforehand cleavage of HCV RNA at 37°C and the single-stranded oligo DNA (promoter primer) designed for amplification of HCV RNA at a constant temperature of 50°C. Thus, it was demonstrated that appropriate combined use of single-stranded oligo DNAs of the present invention enables RNA amplification at constant temperature.

As described above, the present invention provides a single-stranded oligo DNA complementary to any single-stranded RNA inclusive of HCV RNA which binds to the RNA with high binding ability. The oligo DNA is complementary to a conformationally free region in the single-stranded RNA such as HCV RNA and facilitates amplification of the RNA.

The present invention also provides a method of preparing the above-mentioned single-stranded oligo DNA. In the method, an arbitrary single-stranded RNA is firstly cleaved by treatment with a randomized multiple single-stranded oligo DNA, and the cleavage products are analyzed to estimate conformationally free regions. Use of a randomized multiple single-stranded oligo DNA of about 6 nucleotides long favorably enables simultaneous assessment of all the possible randomized oligo DNA sequences. Then, for the purpose of imparting binding specificity for the RNA to the finally obtainable single-stranded oligo DNA, sequences of 20 to 25 nucleotides complementary to sequences in the RNA covering an estimated conformationally free region are prepared and assessed similarly, and thus a single-stranded oligo DNA which binds to the single-stranded RNA more firmly with higher binding specificity becomes available.

Thus, according to the present invention, it is possible to provide a single-stranded oligo DNA which firmly binds to a single-stranded RNA as a possible important element for designing a promoter primer used for cleavage, amplification and detection of the RNA or an inhibitor of reverse transcription and translation of the RNA without difficulties in a short time.

## Claims

1. An HCV RNA-binding single-stranded oligo DNA of SEQ ID NO:1 which is complementary to nucleotides 101 to 120 of HCV RNA.

2. An HCV RNA-binding single-stranded oligo DNA of SEQ ID NO:2 which is complementary to nucleotides 99 to 118 of HCV RNA.

3. An HCV RNA-binding single-stranded oligo DNA of SEQ ID NO:3 which is complementary to nucleotides 97 to 116 of HCV RNA.

4. An HCV RNA-binding single-stranded oligo DNA of SEQ ID NO:4 which is complementary to nucleotides 95 to 114 of HCV RNA.

5. An HCV RNA-binding single-stranded oligo DNA of SEQ ID NO:5 which is complementary to nucleotides 248 to 267 of HCV RNA.

6. An HCV RNA-binding single-stranded oligo DNA of SEQ ID NO:6 which is complementary to nucleotides 238 to 257 of HCV RNA.

7. A single-stranded oligo DNA of SEQ ID NO:7 which is useful as a sense primer or a promoter primer and is identical to nucleotides 113 to 137 of HCV RNA.

8. A single-stranded oligo DNA of SEQ ID NO:8 which is useful as a sense primer or a promoter primer and is identical to nucleotides 115 to 139 of HCV RNA.

9. A single-stranded oligo DNA of SEQ ID NO:9 which is useful as a sense primer or a promoter primer and is identical to nucleotides 111 to 135 of HCV RNA.

10. A method of preparing a single-stranded oligo DNA which binds to a single-stranded RNA, which comprises estimating a cleaving site in the single-stranded RNA by experimenting with cleavage reactions using a randomized single-stranded oligo DNA of 6 to 9 nucleotides long and an enzyme which selectively cleaves a heteroduplexed RNA and screening a multiple oligo DNA set of 20 to 25 nucleotides long containing a sequence complementary to the estimated cleaving site or its vicinity by experimenting with cleavage reactions using the enzyme which selectively cleaves a heteroduplexed RNA.

11. A reagent for cleavage of a single-stranded RNA, amplification of a single-stranded RNA, reverse transcription of a single-stranded RNA, inhibition of translation of a single-stranded RNA or assay of a single-stranded RNA, which is part of the single-stranded oligo DNA according to any one of Claims 1 to 6, the whole of the single-stranded oligo DNA according to any one of Claims 1 to 6, part of the single-stranded oligo DNA selected by the method according to Claim 10, the whole of the single-stranded oligo DNA selected by the method according to Claim 10 or a derivative thereof.

12. A reagent for cleaving a single-stranded RNA, which comprises an RNA-cleaving motif and part of the single-stranded oligo DNA according to any one of Claims 1 to 6, the whole of the single-stranded oligo DNA according to any one of Claims 1 to 6, part of the single-stranded oligo DNA selected by the method according to Claim 10, the whole of the single-stranded oligo DNA selected by the method according to Claim 10 or a derivative thereof linked to the RNA-cleaving motif.

13. A reagent as a sense primer or promoter primer for a single-stranded RNA, which is a single-stranded oligo DNA complementary to part of the single-stranded oligo DNA according to any one of Claims 1 to 6, the whole of the single-stranded oligo DNA according to any one of Claims 1 to 6, part of the single-stranded oligo DNA selected by the method according to Claim 10 or the whole of the single-stranded oligo DNA selected by the method according to Claim 10 or a derivative thereof.


SEQUENCE LISTING
<110> Tosoh Corporation
<120> HCV RNA-Binding oligo DNA and simple method of
preparing the DNA
<130> PA210-9629
<160> 14
<210> 1
<211> 20
<212> DNA
<213> Artificial Sequence
<223> Complementary to nucleotides 101 to 120 in HCV RNA
<400> 1
ccgggagggg gggtcctgga 20
<210> 2
<211> 20
<212> DNA
<213> Artificial Sequence
<223> Complementary to nucleotides 99 to 118 in HCV RNA
<400> 2
gggagggggg gtcctggagg 20
<210> 3
<211> 20
<212> DNA
<213> Artificial Sequence
<223> Complementary to nucleotides 97 to 116 in HCV RNA
<400> 3
   gagggggggt cctggaggct 20

<210> 4

<211> 20

<212> DNA

<213> Artificial Sequence

<223> Complementary to nucleotides 95 to 114 in HCV RNA

<400> 4

gggggggtcc tggaggctgc                                        20

<210> 5

<211> 20

<212> DNA

<213> Artificial Sequence

<223> Complementary to nucleotides 248 to 267 in HCV RNA

<400> 5

gcctttcgcg acccaacact                                        20

<210> 6

<211> 20

<212> DNA

<213> Artificial Sequence

<223> Complementary to nucleotides 238 to 257 in HCV RNA

<400> 6

acccaacact actcggctag                                        20

<210> 7

<211> 25

<212> DNA

<213> Artificial Sequence

<223> Identical to nucleotides 113 to 137 in HCV RNA

<400> 7

cctcccggga gagccatagt ggtct 25

<210> 8

<211> 25

<212> DNA

<213> Artificial Sequence

<223> Complementary to nucleotides 115 to 139 in HCV RNA

<400> 8

tcccgggaga gccatagtgg tctgc 25

<210> 9

<211> 25

<212> DNA

<213> Artificial Sequence

<223> Complementary to nucleotides 111 to 135 in HCV RNA

<400> 9

cccctcccgg gagagccata gtggt 25

<210> 10

<211> 31

<212> DNA

<213> Artificial Sequence

<223> Containing sequences complementary to nucleotides

99 to 106 and 108 to 115 in HCV RNA

<400> 10

aggggggggg ctagctacaa cgacctggag g 31

<210> 11

<211> 52

<212> DNA

<213> Artificial Sequence

<223> Promoter primer

<400> 11

atttaggtga cactatagaa tacaacctcc cgggagagcc atagtggtct

50

<210> 12

<211> 50

<212> DNA

<213> Artificial Sequence

<223> Promoter primer

<400> 12

atttaggtga cactatagaa tacaatcccg ggagagccat agtggtctgc

50

<210> 13

<211> 50

<212> DNA

<213> Artificial Sequence

<223> Promoter primer

<400> 13

atttaggtga cactatagaa tacaaccggg agagccatag tggtctgcgg

50

<210> 14

<211> 50

<212> DNA

<213> Artificial Sequence

<223> Promoter primer

<400> 14

atttaggtga cactatagaa tacaacccct cccgggagag ccatagtggt

50